# EUROPEAN PATENT APPLICATION

(11) **EP 4 245 310 A1**
(43) Date of publication of application: **20.09.2023**
(21) Application number: 21892387.8
(22) Date of filing: 15.11.2021
(51) Int. Cl.: A61K 38/00, A61K 38/47, A61K 47/68, A61P 25/00, A61P 25/02

(54) **USE OF THERAPEUTIC ENZYME FUSION PROTEIN IN PREVENTING AND TREATING NEUROPATHY ATTRIBUTED TO OR ASSOCIATED WITH FARBRY'S DISEASE**

(30) Priority: 13.11.2020 KR 20200152246
(71) Applicant: Hanmi Pharm. Co., Ltd., Hwaseong-si, Gyeonggi-do 18536 (KR)
(72) Inventor: KIM, Jin Young, Hwaseong-si, Gyeonggi-do 18469 (KR); PARK, Cho Rong, Hwaseong-si, Gyeonggi-do 18469 (KR); KIM, Sang Yun, Hwaseong-si, Gyeonggi-do 18469 (KR); KIM, Won Ki, Hwaseong-si, Gyeonggi-do 18469 (KR); PARK, Su Yeon, Hwaseong-si, Gyeonggi-do 18469 (KR)
(74) Representative: Schiweck Weinzierl Koch Patentanwälte Partnerschaft mbB
(86) International application number: PCT/KR2021/016629
(87) International publication number: WO 2022/103220

(57) **Abstract**

The present invention relates to use of a fusion protein of a therapeutic enzyme and an immunoglobulin Fc region in the prevention or improvement of neuropathy.

## Description

### [Technical Field]

The present invention relates to use of an enzyme fusion protein including a therapeutic enzyme in the prevention and treatment of neuropathy caused by or accompanied by Fabry disease.

### [Background Art]

Neuropathy is a disease in which functional disorders or pathological changes occur in the nervous system due to various reasons such as diabetes, trauma, autoimmune diseases, renal diseases, tumor, drinking, hereditary disorder, etc. Fabry disease is an inherited disease that causes neuropathy and is one of lysosomal storage disorders. It is known to be caused by accumulated Gb3 and lyso-Gb3 due to deficiency or lack of the enzymatic activity of alpha-galactosidase, which affects the nervous system, resulting in various peripheral nerve symptoms (e.g., polyneuropathy, small fiber neuropathy, neuropathic pain).

Alpha-galactosidase is an enzyme that breaks down Gb3 (globotriaosylceramide) and lyso-Gb3 into lactosylceramide. Abnormalities in this enzyme are known to cause an abnormal accumulation of Gb3 and lyso-Gb3 in blood vessel walls and various parts of the body, leading to Fabry disease. When Gb3 and lyso-Gb3 are deposited in cells, especially in clusters of neurons called dorsal root ganglion (DRG), due to defects in alpha-galactosidase, cell deformations such as vacuolation, etc. occur, resulting in sensory and sensorimotor impairment. For this reason, patients with Fabry disease have an elevated threshold for temperature sensation, accompanied by unbearable peripheral neuropathy, autonomic nervous system symptoms, and central nervous system symptoms, such as stabbing or burning.

As a method of treating lysosomal storage diseases including Fabry disease, an enzyme-replacement therapy (ERT) is mainly studied (Frances M. Platt et al., J Cell Biol. 2012 Nov 26;199(5):723-34). In particular, since Fabry disease is caused by a defect in alpha-galactosidase, supplemental treatment with alpha-galactosidase is essential.

However, proteins that exhibit such therapeutic effects are generally easily denatured due to their low stability and are degraded by proteolytic enzymes in the blood. Therefore, they must be frequently administered to patients to maintain their blood levels and activity. However, frequent injections to maintain blood levels cause great pain to the patient. In order to solve these problems, it is necessary to increase the blood stability of a therapeutic agent used in enzyme-replacement therapy and to develop a therapeutic agent that maintains protein activity while maintaining a high drug blood level for a long time.

Accordingly, the importance of developing a therapeutic agent that is capable of treating neuropathy by having a protective effect on peripheral sensory nerves while having high duration in the body is emerging.

### [Disclosure]

### [Technical Problem]

There is a demand for developing a therapeutic agent capable of treating neuropathy by having a protective effect on peripheral sensory nerves.

### [Technical Solution]

An object of the present invention is to provide a pharmaceutical composition for preventing or treating neuropathy caused by or accompanied by Fabry disease, the pharmaceutical composition including an enzyme fusion protein represented by the following Chemical Formula 1: 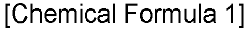 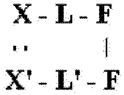
wherein X and X' are each alpha-galactosidase;
L and L' are linkers, each independently the same or a different kind of linker;
F is one polypeptide chain of an immunoglobulin Fc region;
| is a covalent bond; and
: is a covalent or non-covalent bond.

Another object of the present invention is to provide a method of preventing or treating neuropathy caused by or accompanied by Fabry disease, the method including the step of administering the enzyme fusion protein or a composition including the same to an individual in need thereof.

Still another object of the present invention is to provide use of the enzyme fusion protein or the composition including the same in the prevention or treatment of neuropathy caused by or accompanied by Fabry disease.

### [Advantageous Effects]

The present invention relates to use of a fusion protein including a therapeutic enzyme in the prevention or improvement of neuropathy caused by or accompanied by Fabry disease. Due to increased duration of time, the enzyme fusion protein may be usefully applied to patients.

### [Description of Drawings]

FIG. 1 shows the results of comparing therapeutic effects of an alpha-galactosidase-Fc fusion protein (α-galactosidase-Fc) and agalsidase beta on neuropathy; and
FIG. 2 shows the number of vacuolated nerve cells in dorsal root ganglions by the alpha-galactosidase-Fc fusion protein (α-galactosidase-Fc) and agalsidase beta.

### [Best Mode]

One aspect of the present invention provides a pharmaceutical composition for preventing or treating neuropathy caused by or accompanied by Fabry disease, the pharmaceutical composition including an enzyme fusion protein in which a therapeutic enzyme and an immunoglobulin Fc region are fused.

The pharmaceutical composition according to one specific embodiment is characterized in that the enzyme fusion protein is an enzyme fusion protein represented by the following Chemical Formula 1: 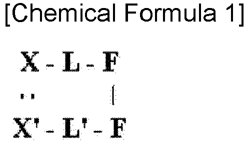
wherein X and X' are each alpha-galactosidase;
L and L' are linkers, each independently the same or a different kind of linker;
F is one polypeptide chain of an immunoglobulin Fc region;
| is a covalent bond; and
: is a covalent or non-covalent bond.

The pharmaceutical composition according to any one of the above specific embodiments is characterized in that the enzyme is an anti-parallel dimer formed by X and X'.

The pharmaceutical composition according to any one of the above specific embodiments is characterized in that the immunoglobulin Fc region is aglycosylated.

The pharmaceutical composition according to any one of the above specific embodiments is characterized in that the immunoglobulin Fc region includes a hinge region having an amino acid sequence of SEQ ID NO: 15.

The pharmaceutical composition according to any one of the above specific embodiments is characterized in that the immunoglobulin Fc region is derived from IgG, IgA, IgD, IgE, or IgM.

The pharmaceutical composition according to any one of the above specific embodiments is characterized in that the immunoglobulin Fc region is derived from an IgG Fc region.

The pharmaceutical composition according to any one of the above specific embodiments is characterized in that the immunoglobulin Fc region is derived from an IgG4 Fc region.

The pharmaceutical composition according to any one of the above specific embodiments is characterized in that the immunoglobulin Fc region is derived from an aglycosylated Fc region derived from human IgG4.

The pharmaceutical composition according to any one of the above specific embodiments is characterized in that the immunoglobulin Fc region is selected from the group consisting of (a) CH1 domain, CH2 domain, CH3 domain, and CH4 domain; (b) CH1 domain and CH2 domain; (c) CH1 domain and CH3 domain; (d) CH2 domain and CH3 domain; and (e) a combination of one domain or two or more domains of CH1 domain, CH2 domain, CH3 domain, and CH4 domain, and an immunoglobulin hinge region or a part of the hinge region.

The pharmaceutical composition according to any one of the above specific embodiments is characterized in that the immunoglobulin Fc region has a substitution of proline for an amino acid at position 2; a substitution of glutamine for an amino acid at position 71; or a substitution of proline for an amino acid at position 2 and a substitution of glutamine for an amino acid at position 71 in an immunoglobulin Fc region having an amino acid sequence of SEQ ID NO: 8.

The pharmaceutical composition according to any one of the above specific embodiments is characterized in that the immunoglobulin Fc region includes a monomer having an amino acid sequence of SEQ ID NO: 9.

The pharmaceutical composition according to any one of the above specific embodiments is characterized in that the linker consists of 1 amino acid to 100 amino acids.

The pharmaceutical composition according to any one of the above specific embodiments is characterized in that the peptide linker consists of an amino acid sequence of [GS]x, [GGGS]x, or [GGGGS]x, wherein x is one natural number of 1 to 20.

The pharmaceutical composition according to any one of the above specific embodiments is characterized in that the peptide linker has an amino acid sequence of SEQ ID NO: 11.

The pharmaceutical composition according to any one of the above specific embodiments is characterized in that the neuropathy is polyneuropathy or small fiber neuropathy.

The pharmaceutical composition according to any one of the above specific embodiments is characterized in that the pharmaceutical composition has a protective effect on peripheral sensory nerves.

The pharmaceutical composition according to any one of the above specific embodiments is characterized in that the pharmaceutical composition inhibits vacuolation of dorsal root ganglion (DRG) cells.

The pharmaceutical composition according to any one of the above specific embodiments is characterized in that the administration frequency of the enzyme fusion protein to an individual in need thereof is reduced as compared to that of an enzyme other than the fusion protein.

The pharmaceutical composition according to any one of the above specific embodiments is characterized in that the pharmaceutical composition is administered to an individual once every 2 weeks or once a month.

The pharmaceutical composition according to any one of the above specific embodiments is characterized in that the enzyme fusion protein includes a monomer including an amino acid sequence of SEQ ID NO: 13.

The pharmaceutical composition according to any one of the above specific embodiments is characterized in that the enzyme fusion protein is a dimer formed by a monomer in which one molecule of the alpha-galactosidase and one molecule of the immunoglobulin Fc region are linked via a peptide linker.

The pharmaceutical composition according to any one of the above specific embodiments is characterized in that X and X' are enzymes, each including an amino acid sequence the same as or different from each other.

Another aspect of the present invention provides a method of preventing or treating neuropathy caused by or accompanied by Fabry disease, the method including the step of administering the enzyme fusion protein or a composition including the same to an individual in need thereof.

Still another aspect of the present invention provides use of the enzyme fusion protein or the composition including the same in the preparation of a prophylactic or therapeutic agent for sequelae of neuropathy caused by or accompanied by Fabry disease.

Still another aspect of the present invention provides use of the enzyme fusion protein or the composition including the same in the prevention or treatment of neuropathy caused by or accompanied by Fabry disease.

### [Mode for Invention]

The present invention will be described in detail as follows. Meanwhile, each description and embodiment disclosed in this disclosure may also be applied to other descriptions and embodiments. That is, all combinations of various elements disclosed in this disclosure fall within the scope of the present invention. Further, the scope of the present invention is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present invention.

Throughout the entire specification, not only the conventional one-letter or three-letter codes for naturally occurring amino acids, but also those three-letter codes generally allowed for other amino acids are used, such as Aib (α-aminoisobutyric acid), Sar (N-methylglycine), etc. Additionally, the amino acids mentioned in abbreviations herein are described according to the IUPAC-IUB rules.

| | |
|---|---|
| Alanine A | Arginine R |
| Asparagine N | Aspartic acid D |
| Cysteine C | Glutamic acid E |
| Glutamine Q | Glycine G |
| Histidine H | Isoleucine I |
| Leucine L | Lysine K |
| Methionine M | Phenylalanine F |
| Proline P | Serine S |
| Threonine T | Tryptophan W |
| Tyrosine Y | Valine V |

One aspect of the present invention provides a pharmaceutical composition for preventing or treating neuropathy caused by or accompanied by Fabry disease, the pharmaceutical composition including an enzyme fusion protein represented by the following Chemical Formula 1: 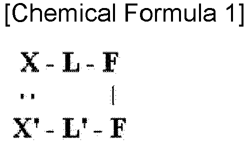
wherein X and X' are each alpha-galactosidase;
L and L' are linkers, each independently the same or a different kind of linker;
F is one polypeptide chain of an immunoglobulin Fc region;
| is a covalent bond; and
: is a covalent or non-covalent bond.

In one embodiment, the pharmaceutical composition may be a pharmaceutical composition including a pharmaceutically acceptable vehicle and the enzyme fusion protein represented by Chemical Formula 1 in a pharmaceutically effective amount. As used herein, the "pharmaceutically effective amount" means a safe dosage of the enzyme fusion protein that does not exhibit toxicity or side effects to patients while exhibiting prophylactic or therapeutic effects on neuropathy caused by or accompanied by Fabry disease, specifically, a dosage having the protective effect on peripheral sensory nerves or obtaining the effect of inhibiting vacuolation of dorsal root ganglion (DRG) cells, but is not limited thereto.

As used herein, the term "enzyme fusion protein" is one in which an immunoglobulin Fc region is fused to a therapeutic enzyme such that the therapeutic enzyme may maintain its activity while its binding affinity for lysosome receptors is reduced, due to fusion of the immunoglobulin Fc region, thereby increasing its blood half-life, as compared to a therapeutic enzyme to which an immunoglobulin Fc region is not fused. The enzyme fusion protein of the present invention may be used as a drug for an enzyme replacement therapy (ERT). The enzyme replacement therapy may prevent or treat a disease through recovery of the deteriorated function of an enzyme by supplementing the defective or deficient enzyme that causes the disease.

The present inventors have prepared a fusion protein with an immunoglobulin Fc region in order to increase the blood half-life of therapeutic enzymes. The Fc region included in the enzyme fusion protein of the present invention may be an IgG4 Fc region, in which a potential glycosylation sequence is substituted to inhibit glycosylation, and additionally, a hinge sequence of IgG4 Fc is substituted to inhibit chain exchange, but is not limited thereto.

In Chemical Formula 1, F which is an immunoglobulin Fc region (e.g., an IgG4 Fc region in which a hinge sequence is substituted) may be linked to a therapeutic enzyme through a linker to form a monomer, and the monomer may form a dimer, together with a monomer including other immunoglobulin Fc region and therapeutic enzyme. With regard to the dimer, the dimer may be formed by a covalent bond between the immunoglobulin Fc regions, and by a covalent or non-covalent bond between the therapeutic enzymes, but is not limited thereto. In particular, it was confirmed that when the therapeutic enzyme to be fused with the immunoglobulin Fc region forms a dimer, particularly, an anti-parallel dimer, *in vivo* duration is increased while maintaining the enzymatic activity. Therefore, the enzyme fusion protein of the present invention has an advantage of increasing stability, as compared to a therapeutic enzyme to which the Fc region is not fused.

As used herein, the term "therapeutic enzyme" refers to an enzyme for treating diseases that occur due to lack, deficiency, malfunction, etc. of enzymes, and refers to an enzyme capable of treating an individual with the diseases through an enzyme replacement therapy, administration, etc. The therapeutic enzyme which may be included in the enzyme fusion protein of the present invention is not particularly limited, and any therapeutic enzyme may be included in the enzyme fusion protein of the present invention, as long as it is a therapeutic enzyme capable of obtaining advantages by longer *in vivo* duration than that of a therapeutic enzyme in an unfused form. In one embodiment of the present invention, the enzyme fusion protein is a fusion protein of a therapeutic enzyme.

In the present invention, the therapeutic enzyme may be alpha-galactosidase.

The therapeutic enzyme included in the enzyme fusion protein of the present invention may form a dimer via a non-covalent bond, but is not limited thereto. Specifically, the therapeutic enzyme may also form a dimer, when the fusion protein is expressed in a transformant and the immunoglobulin Fc region forms a dimer.

Such a dimer of the therapeutic enzymes may be a dimer formed by two enzymes which are the same as each other, or a dimer formed by two enzymes which are different from each other. Specific kinds of the enzymes constituting the dimer are not limited, as long as these enzymes have the desired activity *in vivo.*

Meanwhile, these therapeutic enzymes constituting the dimer may be in the form of a parallel dimer or anti-parallel dimer depending on the direction in which they are connected, but are not limited thereto. For a specific example, with regard to the enzyme in Chemical Formula 1, X and X' may form an anti-parallel dimer, but are not limited thereto.

In one exemplary embodiment of the present invention, a fusion protein was prepared, in which an alpha-galactosidase as the therapeutic enzyme was fused to an immunoglobulin Fc region, and it was confirmed that alpha-galactosidases form an anti-parallel dimer through a non-covalent bond while the immunoglobulin Fc regions of the fusion protein form a dimer (Example 1).

As used herein, the term "parallel dimer" means that the N-terminus and C-terminus of the amino acid sequence of each monomer form a dimer in the same direction when each monomer forms a dimer. In this regard, the dimer may be formed through a non-covalent bond or a covalent bond, but is not limited thereto.

As used herein, the term "anti-parallel dimer" means that the N-terminus and C-terminus of the amino acid sequence of each monomer form a dimer in a different direction from each other when each monomer forms a dimer. In this regard, the dimer may be formed through a non-covalent bond or a covalent bond, but is not limited thereto.

That is, in the enzyme fusion protein of the present invention, it is possible that (i) the N-terminus of one therapeutic enzyme (X) and the N-terminus of another therapeutic enzyme (X') may form a dimer in the same direction, (ii) the C-terminus of one therapeutic enzyme (X) and the C-terminus of another therapeutic enzyme (X') may form a dimer in the same direction, (iii) the N-terminus of one therapeutic enzyme (X) and the C-terminus of another therapeutic enzyme (X') may form a dimer in the same direction, or (iv) the C-terminus of one therapeutic enzyme (X) and the N-terminus of another therapeutic enzyme (X') may form a dimer in the same direction. The dimers in cases (i) and (ii) are called parallel dimers and the dimers in cases (iii) and (iv) are called anti-parallel dimers. The formation of these dimers may occur by a covalent bond or a non-covalent bond, but is not limited thereto.

Specifically, in the formation of the above dimers, the formation of parallel or anti-parallel dimer may be such that, as the immunoglobulin Fc regions form a dimer, the alpha-galactosidase of the monomers linked thereto forms a dimer through a covalent bond or a non-covalent bond.

The enzyme included in the enzyme fusion protein of the present invention may be an alpha-galactosidase. With respect to the objects of the present invention, the enzyme fusion protein may include a dimeric therapeutic enzyme, specifically, a dimer formed by the alpha-galactosidase or a dimer formed by the alpha-galactosidase and another therapeutic enzyme, but is not limited thereto. An example of the fusion protein of the present invention may include a fusion protein, wherein in Chemical Formula 1, X and X' may be alpha-galactosidases, and amino acid sequences of X and X' may be the same as or different from each other. For example, both X and X' may be natural alpha-galactosidases, or any one of X and X' may be a natural alpha-galactosidase, and the other may be a variant in which a part of the sequence of the natural alpha-galactosidase is modified, but are not limited thereto. Alternatively, X and X' may be variants of alpha-galactosidases having different sequences from each other, but are not limited thereto.

As used herein, the term "alpha-galactosidase (α-GAL) or alpha-galactosidase A (α-GAL A)" is an enzyme present in the lysosomes of the spleen, brain, liver, etc., which hydrolyzes terminal alpha-galactosyl moieties in glycolipids and glycoproteins, and is a homodimeric glycoprotein. Specifically, alpha-galactosidase is known to hydrolyze ceramide trihexoside and to catalyze the hydrolysis of melibiose into galactose and glucose, and in particular, is known to be associated with Fabry disease, which is a lysosomal storage disease.

In the present invention, the alpha-galactosidase may include a recombinant form of agalsidase alpha or agalsidase beta, and any enzyme may be included in the scope of the present invention without limitation in its sequence, origin, preparation method, etc., as long as the enzyme is an enzyme exhibiting the activity and therapeutic effect equivalent thereto. Specifically, the alpha-galactosidase may be encoded by a polynucleotide sequence of SEQ ID NO: 5, and may include or may (essentially) consist of an amino acid sequence of SEQ ID NO: 6, but is not limited thereto. Alternatively, the alpha-galactosidase of the present invention may include an amino acid sequence having 60%, 70%, 80% or more, 90% or more, more specifically, 91%, 92%, 93%, 94%, 95%, 96%, 97%, 98%, or 99% or more homology to the natural alpha-galactosidase or the amino acid sequence of SEQ ID NO: 6, but is not limited thereto.

Even though it is described as a peptide 'consisting of' a specific sequence number in the present invention, as long as a peptide has an activity identical or equivalent to that of the peptide consisting of the amino acid sequence of the corresponding sequence number, it is apparent that addition of a meaningless sequence before or after of the amino acid sequence of the corresponding sequence number, a naturally occurring mutation, or a silent mutation thereof are not excluded, and the sequence addition or mutation is included in the scope of the present invention. In other words, even though there is a difference in some sequences, as long as it exhibits homology at a predetermined level or more and exhibits activity equivalent or similar to that of the natural alpha-galactosidase, it may fall within the scope of the present invention.

As used herein, the term 'homology' or 'identity' refers to the degree of relevance between two given amino acid sequences or nucleotide sequences, and may be expressed as a percentage.

The terms 'homology' and 'identity' may be often used interchangeably with each other.

Whether any two peptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity of peptides may be determined by comparing sequence information using, for example, a GAP computer program, for example, Needleman et al. (1970), J Mol Biol. 48:443, as announced in Smith and Waterman, Adv. Appl. Math (1981) 2:482. In summary, the GAP program may be defined as the value acquired by dividing the number of similarly aligned symbols (namely, amino acids) by the total number of symbols in the shorter of two sequences. The default parameters for the GAP program may include (1) a unary comparison matrix (including values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps. Therefore, as used herein, the term "homology" or "identity" refers to the relevance between sequences.

Information on the sequences of the therapeutic enzymes or derivatives thereof and nucleotide sequences encoding the same may be obtained from a known database, e.g., NCBI, etc.

The therapeutic enzyme of the present invention may be a native enzyme, and a fragment consisting of a part of the native enzyme, or an analog of the therapeutic enzyme in which a variation selected from the group consisting of substitution, addition, deletion, and modification of some amino acids, and a combination thereof has occurred, may be included in the present invention without limitation, as long as it has an activity equivalent to that of the native therapeutic enzyme.

As used herein, the term "fragment" refers to a form where one or more amino acids in the amino or carboxy terminus of a native therapeutic enzyme or an analog of the native therapeutic enzyme are removed. Any fragment belongs to the scope of the present invention regardless of the size of the fragment or the kind of amino acids to be removed, as long as they have the activity of the therapeutic enzyme.

Further, the analog of the therapeutic enzyme includes all of those where one or more amino acids are added to the amino and/or carboxy terminus of the native therapeutic enzyme.

Further, the analog of the therapeutic enzyme may be those in which one or more amino acid residues in the sequence of the native therapeutic enzyme are substituted or added. As amino acids to be substituted or added, not only 20 amino acids commonly found in human proteins, but also atypical or non-naturally occurring amino acids may be used. Commercial sources of the atypical amino acids may include Sigma-Aldrich, ChemPep Inc., Genzyme Pharmaceuticals. The peptides including these amino acids and atypical peptide sequences may be synthesized and purchased from commercial peptide suppliers, e.g., American Peptide Company or Bachem (USA), or Anygen (Korea), but are not particularly limited thereto.

The therapeutic enzyme analogs may include the biosimilars and biobetters of the corresponding therapeutic enzymes. For example, with respect to biosimilars, considering the difference in a host for its expression compared to a known therapeutic enzyme, the difference in glycosylation feature and the degree thereof, and the difference in the degree of substitution in a particular amino acid residue of the corresponding enzyme in light of the standard sequence where the degree of substitution is not 100% substitution, they belong to the biosimilar enzymes which may be used as the enzyme fusion protein of the present invention. The therapeutic enzymes may be prepared or produced by a known method in the art, specifically, by genetic recombination in animal cells, *E. coli,* yeast, insect cells, plant cells, live animals, etc., and the production method is not limited thereto, and commercially available therapeutic enzymes may be purchased and used, but are not limited thereto.

The therapeutic enzyme may be prepared or produced by a method known in the art, and specifically, the enzyme may be purified from the culture after culturing animal cells into which an animal expression vector is inserted, or may be used after purchasing commercially available enzymes, but is not limited thereto.

The enzyme fusion protein of the present invention may be those in which the therapeutic enzyme and the immunoglobulin Fc region are fused via a peptide linker. In other words, L or L' in Chemical Formula 1 may be a peptide linker, but is not particularly limited, as long as it is able to fuse the immunoglobulin Fc region with the therapeutic enzyme.

The peptide linker may include one or more amino acids, for example, 1 amino acid to 1000 amino acids, 1 amino acid to 500 amino acids, 1 amino acid to 100 amino acids, or 1 amino acid to 50 amino acids, and any peptide linker known in the art, e.g., including [GS]x linker, [GGGS]x linker, and [GGGGS]x linker, etc., wherein x is a natural number of 1 or greater (e.g., 1, 2, 3, 4, 5, or greater), and for specific example, x may be a natural number of 1 to 20, but is not limited thereto. Specifically, the peptide linker of the present invention may consist of 10 to 50 amino acid sequences, more specifically, 20 to 40 amino acid sequences, and may consist of an amino acid sequence of SEQ ID NO: 11.

With respect to the objects of the present invention, the position at which the peptide linker is fused to the therapeutic enzyme and the immunoglobulin Fc is not limited as long as the peptide linker is able to link the therapeutic enzyme and the immunoglobulin Fc while maintaining the activity of the therapeutic enzyme. Specifically, the position may be both ends of the therapeutic enzyme and the immunoglobulin Fc region, and more specifically, the position may be the C-terminus of the therapeutic enzyme and the N-terminus of the immunoglobulin Fc region, but is not limited thereto.

As used herein, the terms "N-terminus" and "C-terminus" refer to an amino end and a carboxyl end of a protein, respectively. For example, the N-terminus or C-terminus may include, but is not limited to, not only the most terminal amino acid residue of the N-terminus or C-terminus, but also the amino acid residues around the N-terminus or C-terminus, and specifically, the 1^{st} amino acid residue to the 20^{th} amino acid residue from the most terminus.

In one exemplary embodiment of the present invention, a fusion protein (SEQ ID NO: 13), in which the N-terminus of IgG4 Fc region is fused to the C-terminus of the therapeutic enzyme, was prepared via synthesis such that alpha-galactosidase as the therapeutic enzyme and a linker-IgG4 are fused at a gene level, and it was confirmed that the fusion protein is expressed in a transformant into which the fusion protein is transformed (Example 1).

For one specific example, the enzyme fusion protein of the present invention may include a monomer which is formed by linking the alpha-galactosidase to the immunoglobulin Fc region by a covalent bond via the peptide linker, wherein a dimer may be formed by a covalent bond between the immunoglobulin Fc regions and by a covalent or non-covalent bond between the therapeutic enzymes in the two monomers, but is not limited thereto.

For another specific example, the enzyme fusion protein of the present invention may include a monomer including or (essentially) consisting of an amino acid sequence of SEQ ID NO: 13, or may have a dimeric structure which is formed by the monomer, but is not limited thereto.

In the present invention, the peptide linkers may be respectively linked to the dimeric immunoglobulin Fc region, which is formed by the monomeric immunoglobulin Fc regions, in which the linkers linked to respective immunoglobulin Fc regions may be the same as or different from each other.

As used herein, the term "immunoglobulin Fc region" refers to a region of an immunoglobulin including the heavy chain constant region 2 (CH2) and/or heavy chain constant region 3 (CH3), excluding heavy and light chain variable regions. With respect to the objects of the present invention, such an Fc region may include a modified hinge region, but is not limited thereto. Specifically, the immunoglobulin Fc region may have a variation selected from the group consisting of substitution, addition, deletion, modification, and a combination thereof in at least one amino acid of a native immunoglobulin Fc region, but is not limited thereto.

Further, in the present invention, F in the enzyme fusion protein of Chemical Formula 1 may be an immunoglobulin Fc region derived from IgG, specifically, IgG4 Fc region, and may be aglycosylated, but is not limited thereto. Further, F may be an immunoglobulin Fc region obtained by substituting one or more amino acids in a human IgG4 Fc region, but is not limited thereto.

In the enzyme fusion protein of Chemical Formula 1, F may be one polypeptide chain of an immunoglobulin Fc region, but is not limited thereto.

Specific examples of F of Chemical Formula 1 may include a monomer in which proline is substituted for an amino acid at position 2; glutamine is substituted for an amino acid at position 71; or proline is substituted for an amino acid at position 2 and glutamine is substituted for an amino acid at position 71 in an immunoglobulin Fc region having an amino acid sequence of SEQ ID NO: 8; or a monomer of SEQ ID NO: 9, but are not limited thereto.

The immunoglobulin Fc region is a material used as a carrier in preparing drugs, and fusion protein studies using an immunoglobulin Fc region have been actively conducted recently so as to stabilize proteins and to prevent them from being removed from the kidneys. Immunoglobulins are major constituents of the blood, and there are five different types such as IgG, IgM, IgA, IgD, and IgE. The most frequently used type for fusion protein studies is IgG, and it is classified into four subtypes of IgG1∼4. Fusion proteins prepared using an immunoglobulin Fc may increase the protein size, thereby preventing their removal in the kidneys, and also bind to FcRn receptors, thereby having a role in increasing the blood half-life through endocytosis and recycling into cells.

In the present invention, the Fc region refers to a natural sequence obtained from papain digestion of an immunoglobulin as well as a derivative thereof, for example, variants having sequences different from the natural form by deletion, insertion, non-conservative or conservative substitution of one or more amino acid residues in the natural sequence, or a combination thereof, provided that the derivatives, substituents, and variants retain the FcRn-binding ability. In the present invention, F may be a human immunoglobulin region, but is not limited thereto. In the enzyme fusion protein of Chemical Formula 1 according to the present invention, F is a monomeric immunoglobulin Fc region including one polypeptide chain, wherein the polypeptide chain forms a dimer of two polypeptide chains due to a disulfide bond, and thus the enzyme fusion protein of the present invention may have a structure including a dimeric immunoglobulin Fc region. In particular, the enzyme fusion protein may have a structure, in which the chains are linked only through a nitrogen atom of one chain of the two chains, but is not limited thereto. The linkage through the nitrogen atom may be linkage through reductive amination at the lysine epsilon amino group or the N-terminal amino group.

The reductive amination reaction means a reaction in which an amine group or an amino group of a reactant reacts with an aldehyde (i.e., a functional group capable of reductive amination) of another reactant to generate an amine, and then to form an amine bond by a reduction reaction, and the reductive amination reaction is an organic synthesis reaction widely known in the art.

In one specific example, the immunoglobulin Fc regions may be linked to each other via a nitrogen atom of the N-terminal proline, but is not limited thereto.

Further, the immunoglobulin Fc region of the present invention may be an extended Fc region including all or part of the heavy chain constant region 1 (CH1) and/or the light constant region 1 (CL1), excluding heavy chain and light chain variable regions of an immunoglobulin, as long as the immunoglobulin Fc region has an effect substantially equivalent to or more improved than that of its native type. Further, the immunoglobulin Fc region of the present invention may be a region in which a significantly long part of an amino acid sequence corresponding to CH2 and/or CH3 is removed.

The immunoglobulin Fc region of the present invention may be 1) CH1 domain, CH2 domain, CH3 domain and CH4 domain, 2) CH1 domain and CH2 domain, 3) CH1 domain and CH3 domain, 4) CH2 domain and CH3 domain, and (5) a combination between one or two or more domains of CH1 domain, CH2 domain, CH3 domain, and CH4 domain and an immunoglobulin hinge region (or a part of the hinge region), but is not limited thereto. More specifically, the immunoglobulin Fc region may consist of a hinge region, a CH2 domain, and a CH3 domain, but is not limited thereto.

In the present invention, F (immunoglobulin Fc region) of Chemical Formula 1 may be in the form of a monomer, but is not limited thereto. Specifically, the immunoglobulin Fc region may include a single-chain immunoglobulin consisting of domains of the same origin, and but is not limited thereto.

In the present invention, the immunoglobulin Fc region which is denoted as F in Chemical Formula 1 may be in the form of a monomer, and may be expressed by a fusion with a monomeric therapeutic enzyme through a peptide linker. As the monomeric immunoglobulin Fc regions form a dimer, the therapeutic enzyme fused to the immunoglobulin Fc region may also form a dimer by a non-covalent bond, but is not limited thereto.

Such an immunoglobulin Fc region may include a hinge region in the heavy chain constant region, and the monomeric immunoglobulin Fc regions may form a dimer due to the hinge region, but is not limited thereto.

In the present invention, the immunoglobulin Fc region may include a specific hinge sequence at the N-terminus.

As used herein, the term "hinge sequence" refers to a site that is located at a heavy chain and forms a dimer of the immunoglobulin Fc region via an inter disulfide bond.

For one example, the hinge sequence may be one in which a part of the hinge sequence having the following amino acid sequence is deleted or modified.
Glu-Ser-Lys-Tyr-Gly-Pro-Pro-Cys-Pro-Ser-Cys-Pro (SEQ ID NO: 14)

Specifically, the hinge region may be one having a variation where a part of the hinge region is deleted to include only one cysteine (Cys) residue; or may be one where a serine (Ser) residue involved in chain exchange is substituted with a proline (Pro) residue, and more specifically, one where the 2^{nd} serine residue of the hinge sequence is substituted with a proline residue, but is not limited thereto.

For one example, the hinge region of the present invention may include or may (essentially) consist of a sequence of Pro-Pro-Cys-Pro (SEQ ID NO: 15), and the immunoglobulin Fc region may include the hinge region sequence of SEQ ID NO: 15, but is not limited thereto.

The immunoglobulin Fc region of the present invention may include a native hinge region or a modified hinge region to form a dimer via a covalent bond of one molecule of the immunoglobulin Fc region, but is not limited thereto.

The immunoglobulin Fc region used as a drug carrier has a disadvantage in that it may cause an unintended immune response, for example, having effector functions such as antibody-dependent cell-mediated cytotoxicity (ADCC) or complement-dependent cytotoxicity (CDC). These functions occur through the binding of an immunoglobulin Fc region to an Fc receptor or complement, or glycosylation of the Fc region. In addition, it is highly likely that instability of Fc itself may occur *in vivo.*

The present inventors have made efforts to solve the above problem by substituting the sequence of the hinge region in the immunoglobulin Fc region. Specifically, the immunoglobulin Fc region of the present invention may be one in which a potent glycosylation sequence is substituted for the regulation of glycosylation or the sequence involved in chain exchange is substituted, or may correspond to both cases. More specifically, the immunoglobulin Fc region of the enzyme fusion protein of the present invention may be one in which no chain exchange occurs.

Specifically, the immunoglobulin Fc region of the present invention may be one in which the 2^{nd} amino acid and/or the 71^{st} amino acid of the immunoglobulin Fc region of SEQ ID NO: 8 is substituted with a different amino acid for the prevention of chain exchange and N-glycosylation. More specifically, the immunoglobulin Fc region of the present invention may be 1) one in which the 2^{nd} amino acid (serine) is substituted with proline, 2) one in which the 71^{st} amino acid (asparagine) is substituted with glutamine, or 3) one in which the 2^{nd} amino acid is substituted with proline and the 71^{st} amino acid is substituted with glutamine in the immunoglobulin Fc region of SEQ ID NO: 8, and specifically, it may be an immunoglobulin Fc region represented by the amino acid sequence of SEQ ID NO: 9, but is not limited thereto. In addition to the variations described above, the immunoglobulin Fc region may include an appropriate variation as a drug carrier for increasing stability of the therapeutic enzyme.

Specifically, the immunoglobulin Fc region may be one in which a hinge region of an immunoglobulin IgG4 Fc is substituted with an IgG1 hinge region, but is not limited thereto.

In one embodiment of the present invention, the 2^{nd} amino acid of the immunoglobulin Fc is substituted with proline and the 71^{st} amino acid of the immunoglobulin Fc is substituted with glutamine, thereby reducing chain exchange and N-glycosylation (Example 1).

As used herein, the term "chain exchange" refers to a problem in that when an IgG4 Fc is used as a carrier of a protein fusion body, the IgG4 Fc forms a hybrid with an IgG4 present *in vivo* or is present as a monomer and alters the original structure to have a structure with a low therapeutic activity, and it was previously reported that there is significant difficulty when a fusion protein body, in which a protein is fused, is used for therapeutic purposes (van der Neut Kolfschoten, et at., Science, 317:1554-1557. 2007).

Further, in another specific embodiment, the immunoglobulin Fc region of the present invention includes not only native amino acid sequences but also sequence analogs thereof. An amino acid sequence analog means that a variation selected from the group consisting of substitution, addition, deletion, modification, and a combination thereof has occurred in one or more amino acid residues of a native amino acid sequence.

For example, amino acid residues at positions 214 to 238, 297 to 299, 318 to 322, or 327 to 331 in IgG Fc, which are known to be important for linkage, may be used as the sites suitable for variation.

Further, various types of analogs are possible, for example, one where the site capable of forming a disulfide bond is removed, one where several N-terminal amino acids from native Fc are removed, one where a methionine residue is added to the N-terminus of native Fc, etc. Further, complement binding sites, e.g., Clq binding sites, or antibody-dependent cell-mediated cytotoxicity (ADCC) sites may be removed in order to remove the effector function. The techniques for preparing sequence analogs of an immunoglobulin Fc region are disclosed in International Publication Nos. WO 97/34631, WO 96/32478, etc.

Amino acid exchange in a protein or peptide molecule that do not alter the entire activity of a molecule are well known in the art (H.Neurath, R.L.Hill, The Proteins, Academic Press, New York, 1979). The most common exchanges occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, etc.

Further, the Fc analogs described above may be those which exhibit the biological activity equivalent to that of the Fc region of the present invention, but which have increased structural stability of the Fc region against heat, pH, etc.

Further, such an Fc region may be obtained from a native type isolated from humans or animals, such as cows, goats, pigs, mice, rabbits, hamsters, rats, guinea pigs, etc., or may be recombinants or analogs thereof obtained from transformed animal cells or microorganisms. Herein, the method of obtaining the Fc region from the native type may be a method of isolating whole immunoglobulins from human or animal organisms and then treating them with a protease. Papain treatment results in the digestion into Fab and Fc, and pepsin treatment results in the digestion into pF'c and F(ab)2. These fragments may be subjected to size exclusion chromatography to isolate Fc or pF'c. In a more specific embodiment, the Fc region may be a recombinant immunoglobulin Fc region where a human-derived Fc region is obtained from a microorganism.

Further, the immunoglobulin Fc region may be in the form of native glycans, increased glycans, as compared to its native type, decreased glycans, as compared to its native type, or in a deglycosylated or aglycosylated form. The increase, decrease, or removal of the immunoglobulin Fc glycans may be achieved by common methods such as a chemical method, an enzymatic method, and a genetic engineering method using a microorganism. Here, the immunoglobulin Fc region where the glycans are removed from the Fc region shows a significant decrease in binding affinity for the complement (Clq) and a decrease or removal of antibody-dependent cytotoxicity or complement-dependent cytotoxicity, and thus it does not induce unnecessary immune responses *in vivo.* In this regard, an immunoglobulin Fc region in a deglycosylated or aglycosylated immunoglobulin Fc region may be a more suitable form to meet the original object of the present invention as a drug carrier.

As used herein, the term "deglycosylation" refers to removal of glycan from an Fc region by an enzyme, and the term "aglycosylation" refers to an unglycosylated Fc region produced in prokaryotes, in a more specific embodiment, in *E*. *coli.*

Meanwhile, the immunoglobulin Fc region may be derived from humans or animals such as cows, goats, pigs, mice, rabbits, hamsters, rats, guinea pigs, etc., and in a more specific embodiment, it may be derived from humans.

Further, the immunoglobulin Fc region may be an Fc region derived from IgG, IgA, IgD, IgE, IgM, or a combination or hybrid thereof. In a more specific embodiment, it may be derived from IgG or IgM, which are the most abundant proteins in human blood, and in an even more specific embodiment, it may be derived from IgG, which is known to enhance the half-lives of ligand-binding proteins. In a more specific embodiment, the immunoglobulin Fc region may be an IgG4 Fc region, in an even more specific embodiment, the immunoglobulin Fc region may be an aglycosylated Fc region derived from a human IgG4, and in the most specific embodiment, an amino acid sequence of the immunoglobulin Fc region is SEQ ID NO: 9 and a polynucleotide sequence encoding the same may be SEQ ID NO: 7, but is not limited thereto.

As used herein, the term "combination" means that polypeptides encoding single-chain immunoglobulin Fc regions of the same origin are linked to a single-chain polypeptide of a different origin to form a dimer or multimer. In other words, a dimer or multimer may be prepared from two or more fragments selected from the group consisting of Fc fragments of IgG Fc, IgA Fc, IgM Fc, IgD Fc and IgE.

Further, the therapeutic enzyme or enzyme fusion protein of the present invention may be those where the N-terminus and/or C-terminus are not modified, but, for protecting the therapeutic enzymes from proteases *in vivo* and increasing stability, those where the N-terminus and/or C-terminus thereof is chemically modified or protected by an organic group, or the peptide terminus is modified by the addition of an amino acid, etc. are also included in the scope of the therapeutic enzyme or enzyme fusion protein according to the present invention. When the C-terminus is not modified, the terminus of the therapeutic enzyme or enzyme fusion protein according to the present invention may have a carboxyl group, but is not particularly limited thereto.

In particular, since the N-terminus and C-terminus of chemically synthesized proteins are charged, the N-terminus may be acetylated and/or the C-terminus may be amidated so as to remove these charges, but are not particularly limited thereto.

As used herein, the term "N-terminus" refers to an amino end of a protein or polypeptide, and may include the most terminal amino acid residue of the amino end, or 1, 2, 3, 4, 5, 6, 7, 8, 9, or 10 or more amino acids from the most terminal end. The immunoglobulin Fc region of the present invention may include a hinge sequence at the N-terminus, but is not limited thereto.

Unless otherwise specified in the present specification, the technologies with regard to "enzyme" or "fusion protein" according to the present invention which are described in the detailed description or claims of the present invention will be applied not only to the corresponding enzyme or fusion protein, but also to the scope which includes all of the salts of the corresponding enzyme or fusion protein (e.g., a pharmaceutically acceptable salt of the fusion protein), or a solvate thereof. Accordingly, although it is simply described as "enzyme" or "fusion protein" in the specification, the corresponding description will likewise be applied to the specific salt, the specific solvate, and the specific solvate of the specific salt. Such salt forms may be in a form of, for example, using any pharmaceutically acceptable salt, but the kind of the salt is not particularly limited. Those salt forms may be, for example, those which are safe and effective to individuals, for example, mammals, but are not particularly limited thereto.

As used herein, the term "pharmaceutically acceptable" refers to a material which may be effectively used for the intended use without causing excessive toxicity, stimulation, or allergic reactions, etc. within the range of medico-pharmaceutical decision.

As used herein, the term "pharmaceutically acceptable salt" refers to a salt derived from pharmaceutically acceptable inorganic salts, organic salts, or bases. Examples of the suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, etc. Examples of the salts derived from suitable bases may include alkali metals such as sodium, potassium, etc., alkali earth metals such as magnesium, ammonium, etc.

Further, as used herein, the term "solvate" refers to a complex formed between a solvent molecule and the enzyme, fusion protein according to the present invention, or a salt thereof.

The enzyme fusion protein of the present invention may be prepared by a method known in the art.

In one embodiment of preparing the enzyme fusion protein of the present invention, a recombinant vector was prepared, where alpha-galactosidase as the therapeutic enzyme may be expressed in a form of being fused to a peptide linker-immunoglobulin Fc, and then expressed in a cell line, but the method is not limited thereto, and the enzyme fusion protein may be prepared by other known methods than the method described herein. The enzyme fusion protein of the present invention may include an amino acid sequence of SEQ ID NO: 13, but is not limited thereto.

The enzyme fusion protein according to the present invention may increase the half-life of the therapeutic enzyme by increasing *in vivo* stability of the therapeutic enzyme while maintaining the activity thereof, via a fusion of the therapeutic enzyme with the immunoglobulin Fc region. In particular, the therapeutic enzyme fused with a modified immunoglobulin Fc region has reduced chain exchange and glycosylation, and thus may have a lower binding affinity for lysosome receptors, as compared to a therapeutic enzyme to which Fc is not fused, thereby having high duration, confirming that such a therapeutic enzyme is effective for the treatment of target diseases.

The above descriptions may also be applied to other specific embodiments or aspects of the present invention, but are not limited thereto.

The pharmaceutical composition of the present invention may be a pharmaceutical composition for preventing or treating neuropathy caused by or accompanied by Fabry disease, the pharmaceutical composition including the enzyme fusion protein in a pharmaceutically effective amount, and optionally, further including a pharmaceutically acceptable excipient. The composition according to the present invention is charactered in that *in vivo* duration and stability of the therapeutic enzyme are increased.

The enzyme fusion protein including alpha-galactosidase according to the present invention may reduce the level of Lyso-Gb3 which is accumulated in the kidney tissue due to the defect of alpha-galactosidase, thereby being used in the treatment of diseases (e.g., neuropathy) caused by Lyso-Gb3 accumulation.

Lyso-Gb3 (Globotriaosylsphingosine) is a deacylated form of Gb3 (globotriaosylceramide), and Lyso-Gb3 or Gb3 is known to play a direct role in the peripheral nociceptive neurons, and the therapeutic effect on neuropathy may be obtained by reducing the level thereof.

The pharmaceutical composition of the present invention may include the enzyme fusion protein to exhibit the prophylactic or therapeutic effect on neuropathy.

As used herein, the term "neuropathy" refers to a disease in which a functional disturbance or pathological change occurs in the nervous system. Depending on the cause, neuropathy may be classified as arsenical neuropathy, diabetic neuropathy, ischemic neuropathy, or traumatic neuropathy. Further, neuropathy may be caused by hereditary diseases, for example, Charcot-Marie-Tooth (CMT), familial amyloidosis, Fabry disease, metachromatic leukodystrophy, etc. In the present invention, neuropathy may be neuropathy caused by or accompanied by Fabry disease.

It is known that, in patients with Fabry disease, Lyso-Gb3 accumulates without being degraded, due to defects in alpha-galactosidase, and the accumulated Lyso-Gb3 affects the nervous system, resulting in many different symptoms of peripheral neuropathy.

Since the enzyme fusion protein of the present invention may be used in the enzyme-replacement therapy, it may exhibit the prophylactic or therapeutic effect on neuropathy caused by or accompanied by Fabry disease due to defects in alpha-galactosidase, or neuropathy that develops due to Fabry disease, and is accompanied by Fabry disease.

With respect to the objects of the present invention, the pharmaceutical composition for preventing or treating neuropathy caused by or accompanied by Fabry disease according to the present invention may exhibit the prophylactic or therapeutic effect on polyneuropathy or small fiber neuropathy, but is not limited thereto. Further, the neuropathy of the present invention may be accompanied by neuropathic pain, but is not limited thereto.

The enzyme fusion protein of the present invention or the pharmaceutical composition including the same may have the protective effect on peripheral sensory nerves and may also inhibit vacuolation of dorsal root ganglion (DRG) cells, but is not limited thereto.

Structural and functional modifications (e.g., vacuolation) of dorsal root ganglion cells are known to induce neuropathy, and the enzyme fusion protein of the present invention may have the effects of preventing and improving sensory and sensorimotor impairment by inhibiting such modifications and protecting nerve cells.

In other words, the enzyme fusion protein of the present invention and the composition including the same may exhibit prophylactic or therapeutic effect on neuropathy caused by or accompanied by Fabry disease by protecting the functions of peripheral sensory nerves and by inhibiting modifications of cells which causes neuropathy.

As used herein, the term "preventing" refers to all activities that inhibit or delay the occurrence of neuropathy caused by or accompanied by Fabry disease by administering the enzyme fusion protein or the composition including the same, and the term "treating" refers to all activities that improve or advantageously change the symptoms of neuropathy caused by or accompanied by Fabry disease by administering the enzyme fusion protein or the composition including the same.

As used herein, the term "administering" refers to the introduction of a particular substance into a patient by any appropriate method, and the administration route of the composition may be, but is not particularly limited to, any common route that enables delivery of the composition to a target *in vivo,* for example, intraperitoneal administration, intravenous administration, intramuscular administration, subcutaneous administration, intradermal administration, oral administration, local administration, intranasal administration, intrapulmonary administration, intrarectal administration, etc. However, since peptides are digested upon oral administration, active ingredients of a composition for oral administration are preferably coated or formulated for protection against degradation in the stomach, and specifically, may be administered in an injectable form. Further, the pharmaceutical composition may be administered using any device capable of transporting the active ingredients into a target cell.

The total effective dose of the composition of the present invention may be administered to a patient in a single dose or may be administered for a long period of time in multiple doses according to a fractionated treatment protocol. In the pharmaceutical composition of the present invention, the content of the active ingredient may vary depending on the disease severity. Specifically, the preferred total daily dose of the fusion protein of the present invention may be about 0.0001 mg to 500 mg per 1 kg of body weight of a patient. However, the effective dose of the fusion protein is determined considering various factors including the patient's age, body weight, health conditions, sex, disease severity, diet, excretion rate, etc., in addition to administration route and treatment frequency of the pharmaceutical composition. In this regard, those skilled in the art may easily determine the effective dose suitable for the particular use of the composition of the present invention. The pharmaceutical composition according to the present invention is not particularly limited to the formulation, administration route, and administration method, as long as it shows the effects of the present invention.

The actual dose of the enzyme fusion protein of the present invention is determined based on the types of the therapeutic enzyme used as an active ingredient, along with various factors, such as the disease to be treated, administration route, a patient's age, sex, and body weight, severity of the disease, etc. Since the enzyme fusion protein of the present invention has very excellent blood duration and *in vivo* activity, the dose, the number and frequency of administration of the pharmaceutical formulation including the enzyme fusion protein of the present invention may be significantly reduced.

Specifically, the enzyme fusion protein of the present invention has the increased duration due to high stability, and may maintain the enzymatic activity for a long period of time, as compared to enzymes that are not in the form of fusion proteins, and therefore, it may exhibit excellent pharmacological effects through continuous exposure to target tissues for treatment.

In one exemplary embodiment of the present invention, it was confirmed that when the enzyme fusion protein was administered to alpha-galactosidase gene knock-out mice by reducing the frequency of administration, the therapeutic effect was equal to or superior to that of the enzyme other than the fusion protein. Accordingly, the frequency of administration of the enzyme fusion protein to an individual in need thereof may be reduced, as compared to that of an enzyme other than the fusion protein, but is not limited thereto.

Unlike the enzyme fusion protein of the present invention, the enzyme that is not in the form of the fusion protein may refer to the enzyme itself to which a carrier (e.g., immunoglobulin Fc region) is not bound. Examples thereof may include Fabrazyme^{®} (agalsidase beta), but are not limited thereto.

Specifically, the enzyme fusion protein according to the present invention may be administered in an amount of about 0.0001 µg to about 500 mg per 1 kg of a patient' body weight, specifically, about 0.001 mg to about 100 mg, specifically, 0.01 mg to 50 mg, and more specifically, 0.1 mg to 10 mg per 1 kg of a patient' body weight once a week, once every two weeks, once every four weeks, or once a month, and even though the administration interval is increased, the pharmacological activity of the enzyme fusion protein is maintained in the body, and therefore, the patient's convenience may be increased.

The pharmaceutical composition according to the present invention may further include a pharmaceutically acceptable carrier, vehicle(excipient), or diluent. Such carriers may be those non-naturally occurring.

As used herein, the term "pharmaceutically acceptable" means an amount sufficient to exhibit the therapeutic effect without causing side effects, and may be easily determined by a person skilled in the art according to factors well known in the medical field, such as the type of disease, a patient's age, body weight, health, sex, and sensitivity of to the drug, an administration route, an administration method, frequency of administration, duration of treatment, drugs blended or simultaneously used, etc.

The pharmaceutically acceptable carrier may include, for oral administration, a binder, a glidant, a disintegrant, an excipient, a solubilizing agent, a dispersant, a stabilizing agent, a suspending agent, a coloring agent, a flavoring agent, etc.; for injections, a buffering agent, a preserving agent, an analgesic, a solubilizing agent, an isotonic agent, a stabilizing agent, etc., which may be used in a mixture; and for topical administrations, a base, an excipient, a lubricant, a preserving agent, etc.

The formulation of the pharmaceutical composition of the present invention may be variously prepared in combination with the pharmaceutically acceptable carrier described above. For example, for oral administration, the pharmaceutical composition may be formulated into tablets, troches, capsules, elixirs, suspensions, syrups, wafers, etc. For injection, the pharmaceutical composition may be formulated into unit-dose ampoules or multi-dose containers. In addition, the pharmaceutical composition may also be formulated into solutions, suspensions, tablets, pills, capsules, sustained-release formulations, etc.

Meanwhile, examples of carriers, excipients, and diluents suitable for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, etc. In addition, the composition may further include a filler, an anticoagulant, a lubricant, a humectant, a flavoring agent, an emulsifier, a preservative, etc.

Further, the enzyme fusion protein may be used by mixing with various carriers approved as pharmaceutical drugs, such as physiological saline or organic solvents. For increasing stability or absorptivity, carbohydrates such as glucose, sucrose, or dextrans, and antioxidants such as ascorbic acid and glutathione, chelating agents, low molecular weight proteins, or other stabilizers may be used as pharmaceutical drugs.

The pharmaceutical composition may include, but is not limited to, the above ingredients (active ingredients) in an amount of 0.01 % to 99% (w/v).

Another aspect of the present invention provides a method of preventing or treating neuropathy caused by or accompanied by Fabry disease, the method including the step of administering the enzyme fusion protein or the composition including the same to an individual.

The enzyme fusion protein, the composition, the neuropathy, the preventing and treating are the same as described above.

Since the enzyme fusion protein of the present invention may include the therapeutic enzyme capable of preventing or treating neuropathy caused by or accompanied by Fabry disease, neuropathy caused by or accompanied by Fabry disease may be prevented or treated in an individual suspected of having the disease by administering the enzyme fusion protein, or the pharmaceutical composition including the enzyme fusion protein.

As used herein, the term "individual" refers to an individual suspected of having neuropathy, and the individual suspected of having neuropathy refers to mammals including mice, livestock, etc., including humans who already have the corresponding disease or may develop the disease, but the individual includes any individuals without limitation, as long as they may be treated with the enzyme fusion protein of the present invention or the composition including the same. In particular, the individual may be an individual who has developed Fabry disease or at a high risk of Fabry disease, but any individual is included in the scope of the present invention without limitation, as long as the individual has developed or may develop neuropathy associated with Fabry disease.

The method of present invention may include administering a pharmaceutically effective amount of the pharmaceutical composition including the enzyme fusion protein. An appropriate total daily dose may be determined within the scope of correct medical judgment by a practitioner, and the composition may be administered once or several times in divided doses. However, with respect to the objects of the present invention, preferably, the specific therapeutically effective dose for any particular patient is applied differently depending on various factors including the kind and degree of responses to be achieved, specific compositions including whether other agents are occasionally used therewith, the patient's age, body weight, health conditions, sex and diet, administration time, administration route, excretion rate of the composition, duration of treatment, other drugs used in combination or simultaneously with the specific compositions, and similar factors well known in the medical field.

The administration route, the administration dose, and the usage are the same as described above.

Meanwhile, the method of preventing or treating the neuropathy may be a combination therapy which further includes administering one or more compounds or materials having a therapeutic activity on the neuropathy, but the method is not limited thereto.

As used herein, the term "combination" must be understood as referring to a simultaneous, separate, or sequential administration. When the administration is sequential or separate, the interval allowed for the administration of a second ingredient must be one which should not lose the advantageous effects of the combination.

Still another aspect of the present invention provides use of the enzyme fusion protein or the composition including the same in the prevention or treatment of neuropathy caused by or accompanied by Fabry disease.

The enzyme fusion protein, the composition, the neuropathy, the preventing and treating are the same as described above.

Still another aspect of the present invention provides use of the enzyme fusion protein or the composition including the same in the preparation of a prophylactic or therapeutic agent (or a pharmaceutical composition) for neuropathy caused by or accompanied by Fabry disease.

The enzyme fusion protein, the composition, the neuropathy, the preventing and treating are the same as described above.

Hereinafter, the present invention will be described in more detail with reference to exemplary embodiments. However, these exemplary embodiments are only for illustrative purposes only, and the scope of the present invention is not intended to be limited by these exemplary embodiments.

### Example 1: Preparation of alpha-galactosidase-Fc fusion protein (α-galactosidase-Fc)

To produce an alpha-galactosidase-Fc fusion protein (hereinafter, used interchangeably with an enzyme fusion protein) including a therapeutic enzyme in the form of an anti-parallel dimer, the present inventors have fused a native alpha-galactosidase, a linker (SEQ ID NO: 10), and an Fc immunoglobulin region (SEQ ID NO: 7) at the gene level, and have inserted the product into an expression vector.

To remove the sites for chain exchange and N-glycosylation in the Fc regions of the sequence of the constructed enzyme fusion protein, a site-directed mutagenesis PCR technique was used.

In detail, serine which is the 2^{nd} amino acid of the Fc region (SEQ ID NO: 8) involved in the chain exchange was substituted with proline using primers of SEQ ID NOS: 1 and 2, and asparagine which is the 71^{st} amino acid of the Fc region involved in the N-glycosylation was substituted with glutamine using primers of SEQ ID NOS: 3 and 4.

**[Table 1]**

| **Mutagenesis primer** | | |
|---|---|---|
| **Primer** | **Sequence** | SEQ ID NO: |
| Fc(S2P)_F | 5'- CTGGOGGTGGCGGATCGCCACCATGCAGCACCTGAGTTCCT-3' | 1 |
| Fc(S2P)_R | 5'- AGGAACTCAGGTGCTGGGCATGGTGGCGATOOGOCACCGOCAG-3' | 2 |
| Fc(N71Q)_F | 5'- AGCCGOGGGAGGAGCAGTTCCAAAGCACGTAOOGTGTGGTCAG-3' | 3 |
| Fc(N71Q)_R | 5'- CTGACCACACGGTACGTGCTTIGGAACTGCTCCTCCCGCGGCT-3' | 4 |

The polynucleotide encoding the synthesized alpha-galactosidase-Fc fusion protein was inserted into an XOGC vector, which is an expression vector, using restriction enzymes. Both BamHI and Xhol are restriction enzymes that do not cleave alpha-galactosidase and Fc immunoglobulin regions. The alpha-galactosidase-Fc cleaved with the above restriction enzymes was inserted into the XOGC vector cleaved with the same restriction enzymes, thereby completing a vector capable of expressing an alpha-galactosidase-Fc fusion protein. The alpha-galactosidase forms an anti-parallel dimer when the immunoglobulin Fc regions form a dimer.

The DNA and protein sequences of alpha-galactosidase-Fc are as in Table 2 below. In Table 2 below, the underlined parts of the protein sequences represent a signal sequence, the bold parts represent substituted amino acids, and the italic parts represent linkers.

**[Table 2]**

| Name | **Sequence** | | **SEQ ID NO:** |
|---|---|---|---|
| **Alpha-galactosidase -Fc** | DNA | ATGCAGCTGA GGAACCCAGA ACTACATCTG GGCTGCGCGC TTGCGCTTCG CTTCCTGGCC CTCGTTTCCT GGGACATCCC TGGGGCTAGA GCACTGGACA ATGGATTGGC AAGGACGCCT ACCATGGGCT GGCTGCACTG GGAGCGCTTC ATGTGCAACC TTGACTGCCA GGAAGAGCCA GATTCCTGCA TCAGTGAGAA GCTCTTCATG GAGATGGCAG AGCTCATGGT CTCAGAAGGC TGGAAGGATG CAGGTTATGA GTACCTCTGC ATTGATGACT GTTGGATGGC TCCCCAAAGA GATTCAGAAG GCAGACTTCA GGCAGACCCT CAGCGCTTTC CTCATGGGAT TCGCCAGCTA GCTAATTATG TTCACAGCAA AGGACTGAAG CTAGGGATTT ATGCAGATGT TGGAAATAAA ACCTGCGCAG GCTTCCCTGG GAGTTTTGGA TACTACGACA TTGATGCCCA GACCTTTGCT GACTGGGGAG TAGATCTGCT AAAATTTGAT GGTTGTTACT GTGACAGTTT GGAAAATTTG GCAGATGGTT ATAAGCACAT GTCCTTGGCC CTGAATAGGA CTGGCAGAAG CATTGTGTAC TCCTGTGAGT GGCCTCTTTA TATGTGGCCC TTTCAAAAGC CCAATTATAC AGAAATCCGA CAGTACTGCA ATCACTGGCG AAATTTTGCT GACATTGATG ATTCCTGGAA AAGTATAAAG AGTATCTTGG ACTGGACATC TTTTAACCAG GAGAGAATTG TTGATGTTGC TGGACCAGGG GGTTGGAATG ACCCAGATAT GTTAGTGATT GGCAACTTTG GCCTCAGCTG GAATCAGCAA GTAACTCAGA TGGCCCTCTG GGCTATCATG GCTGCTCCTT TATTCATGTC TAATGACCTC CGACACATCA GCCCTCAAGC CAAAGCTCTC CTTCAGGATA AGGACGTAAT TGCCATCAAT CAGGACCCCT TGGGCAAGCA AGGGTACCAG CTTAGACAGG GAGACAACTT TGAAGTGTGG GAACGACCTC TCTCAGGCTT AGCCTGGGCT | 12 |
| | | GTAGCTATGA TAAACCGGCA GGAGATTGGT GGACCTCGCT CTTATACCAT CGCAGTTGCT TCCCTGGGTA AAGGAGTGGC CTGTAATCCT GCCTGCTTCA TCACACAGCT CCTCCCTGTG AAAAGGAAGC TAGGGTTCTA TGAATGGACT TCAAGGTTAA GAAGTCACAT AAATCCCACA GGCACTGTTT TGCTTCAGCT AGAAAATACA ATGCAGATGT CATTAAAAGA CTTACTTGGC GGCGGAGGTT CAGGTGGTGG TGGCTCTGGC GGTGGAGGGT CGGGGGGAGG CGGCTCTGGA GGAGGGGGCT CGGGTGGGGG AGGTAGCCCA CCATGCCCAG CACCTGAGTT CCTGGGGGGA CCATCAGTCT TCCTGTTCCC CCCAAAACCC AAGGACACCC TCATGATCTC CCGGACCCCT GAGGTCACAT GCGTGGTGGT GGACGTGAGC CAGGAAGACC CTGAGGTCCA GTTCAACTGG TACGTGGACG GCGTGGAGGT GCATAATGCC AAGACAAAGC CGCGGGAGGA GCAGTTCCAA AGCACGTACC GTGTGGTCAG CGTCCTCACC GTCCTGCACC AGGACTGGCT GAATGGCAAG GAGTACAAGT GCAAGGTCTC CAACAAAGGC CTCCCATCCT CCATCGAGAA AACCATCTCC AAAGCCAAAG GGCAGCCCCG AGAACCACAG GTGTACACCC TGCCCCCATC CCAGGAGGAG ATGACCAAGA ACCAGGTCAG CCTGACCTGC CTGGTCAAAG GCTTCTATCC CAGCGACATC GCCGTGGAGT GGGAGAGCAA TGGGCAGCCG GAGAACAACT ACAAGACCAC GCCTCCCGTG CTGGACTCCG ACGGCTCCTT CTTCCTCTAC AGCAGGCTAA CCGTGGACAA GAGCAGGTGG CAGGAGGGGA ACGTCTTCTC ATGCTCCGTG ATGCATGAGG CTCTGCACAA CCACTACACG CAGAAGAGCC TCTCCCTGTC TCTGGGTAAA TGA | |
| | Protein | MQLRNPELHL GCALALRFLA LVSWDIPGAR ALDNGLARTP TMGWLHWERF MCNLDCQEEP DSCISEKLFM EMAELMVSEG WKDAGYEYLC IDDCWMAPQR DSEGRLQADP QRFPHGIRQL ANYVHSKGLK LGIYADVGNK TCAGFPGSFG YYDIDAQTFA DWGVDLLKFD GCYCDSLENL ADGYKHMSLA LNRTGRSIVY | 13 |
| | | SCEWPLYMWP FQKPNYTEIR QYCNHWRNFA DIDDSWKSIK SILDWTSFNQ ERIVDVAGPG GWNDPDMLVI GNFGLSWNQQ VTQMALWAIM AAPLFMSNDL RHISPQAKAL LQDKDVIAIN QDPLGKQGYQ LRQGDNFEVW ERPLSGLAWA VAMINRQEIG GPRSYTIAVA SLGKGVACNP ACFITQLLPV KRKLGFYEWT SRLRSHINPT GTVLLQLENT MQMSLKDLLG GGGSGGGGSG GGGSGGGGSG GGGSGGGGSP PCPAPEFLGG PSVFLFPPKP KDTLMISRTP EVTCVVVDVS QEDPEVQFNW YVDGVEVHNA KTKPREEQFQ STYRVVSVLT VLHQDWLNGK EYKCKVSNKG LPSSIEKTIS KAKGQPREPQ VYTLPPSQEE MTKNQVSLTC LVKGFYPSDI AVEWESNGQP ENNYKTTPPV LDSDGSFFLY SRLTVDKSRW QEGNVFSCSV MHEALHNHYT QKSLSLSLGK | |

The enzyme fusion protein-expressing vector prepared in this Example was named alpha-galactosidase-Fc.

### Example 2: In vivo neuropathy-improving Effect of alpha-galactosidase-Fc fusion protein (α-galactosidase-Fc)

In order to confirm the effect of the enzyme fusion protein of the present invention on sensory and sensorimotor impairment of the peripheral nervous system including pain and loss of temperature sensation due to accumulation of Lyso-Gb3, the following experiment was conducted.

In order to evaluate the therapeutic effect on neuropathy according to administration of the alpha-galactosidase-Fc fusion protein (α-galactosidase-Fc) to alpha-galactosidase gene knock-out mice (α-Gal A KO, Jackson laboratory Stock No. 003535), 5 groups of mice were prepared. A first group included wild-type mice (n=6) to which a vehicle of alpha-galactosidase-Fc fusion protein was administered. A second group to the rest are alpha-galactosidase gene knock-out mouse (n=7) experimental groups; Group-2: alpha-galactosidase-Fc fusion protein vehicle administered, Group-3: 1.0 mg/kg of agalsidase beta (once every 2 weeks, intravenous administration), Group-4: 1.0 mg/kg of alpha-galactosidase-Fc fusion protein (once every 2 weeks, subcutaneous administration), and Group-5: 2.0 mg/kg of alpha-galactosidase-Fc fusion protein (once every 4 weeks, subcutaneous administration).

To evaluate sensation of the peripheral nervous system, the degree of improvement in temperature sensation was evaluated by performing a hot plate test on mice before administration, 12 weeks after administration, and 20 weeks after administration, respectively. These animals were placed in a glass cylinder on a hot plate adjusted to a temperature of 55°C, the behaviors of the mice were observed, and the reaction time (latency time) until the hind paws came off the hot plate was measured.

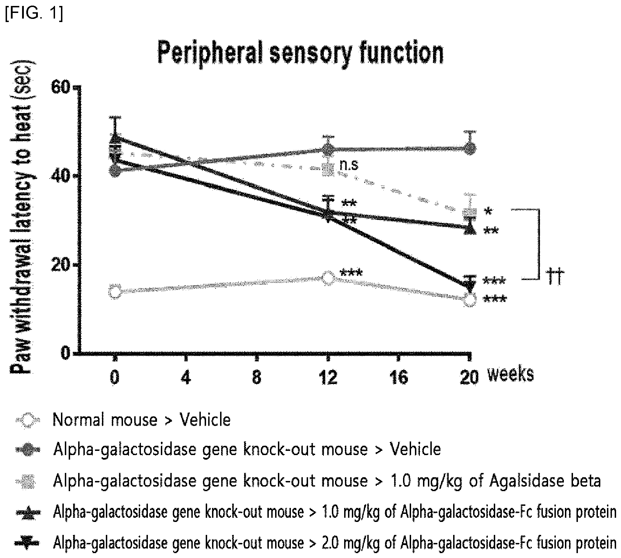

As a result, alpha-galactosidase gene knock-out mice showed a much higher threshold latency in the hot plate test, as compared to wild-type mice. Upon repeated administration of the alpha-galactosidase-Fc fusion protein, they showed a lower threshold latency, as compared to the vehicle control group and the agalsidase beta-administered group. In particular, it was confirmed that the threshold latency in mice repeatedly administered with the alpha-galactosidase-Fc fusion protein was significantly lowered to a level statistically comparable to that of the wild-type mice (FIG. 1).

In addition, at 20 weeks, the dorsal root ganglion (DRG), where the nerve cell bodies of sensory neurons are located from the lumbar spine, was stained with toluidine blue, and histologically analyzed to evaluate the neuropathy improvement effect of the alpha-galactosidase-Fc fusion protein at the cellular level.

Statistical analysis of the two experiments was performed using one-way ANOVA (* - ***p<0.05 - 0.001) and unpaired T test (††p < 0.01) to compare a statistical significance between the vehicle group (control group) and the experimental groups.

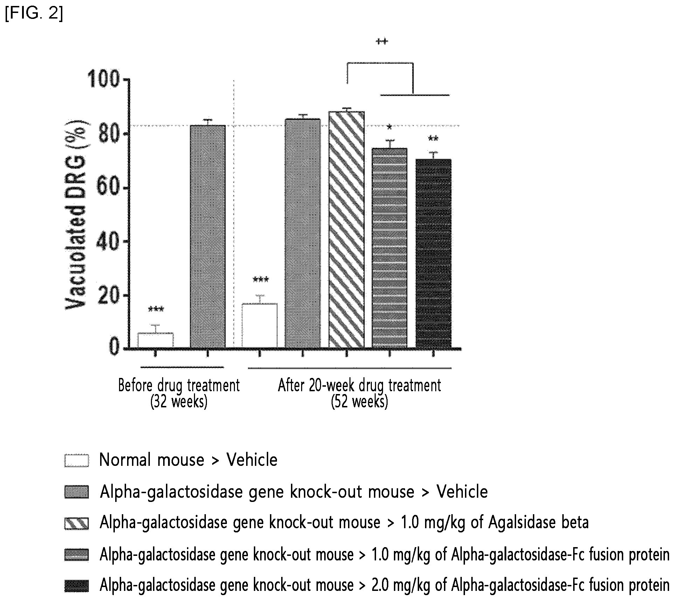

As a result, in the histological analysis at the cellular level, it was confirmed that alpha-galactosidase gene knock-out mice showed a superior increase of the vacuolated neurons in the dorsal root ganglion, as compared to the wild-type mice, which is consistent with the results of the hot plate test of FIG. 1. It was confirmed that these cellular lesions were suppressed at a statistically significant level in the mouse group administered with the alpha-galactosidase-Fc fusion protein for 20 weeks. However, the agalsidase beta-administered group showed no significant improvement, as compared to the vehicle-administered group (FIG. 2).

These experimental results imply that the alpha-galactosidase-Fc fusion protein has improved stability in lysosomes, and based on this, it may exhibit protective or therapeutic effects on neuropathy through protection of peripheral sensory nerves and inhibition of nerve cell deformation.

Based on the above description, it will be understood by those skilled in the art that the present invention may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A pharmaceutical composition for preventing or treating neuropathy caused by or accompanied by Fabry disease, the pharmaceutical composition comprising an enzyme fusion protein represented by the following Chemical Formula 1: 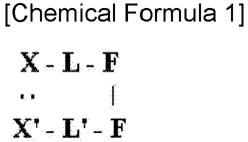
wherein X and X' are each alpha-galactosidase;
L and L' are linkers, each independently the same or a different kind of linker; F is one polypeptide chain of an immunoglobulin Fc region;
| is a covalent bond; and
: is a covalent or non-covalent bond.

2. The pharmaceutical composition of claim 1, wherein the enzyme is an anti-parallel dimer formed by X and X'.

3. The pharmaceutical composition of claim 1, wherein the immunoglobulin Fc region is aglycosylated.

4. The pharmaceutical composition of claim 1, wherein the immunoglobulin Fc region includes a hinge region having an amino acid sequence of SEQ ID NO: 15.

5. The pharmaceutical composition of claim 1, wherein the immunoglobulin Fc region has a substitution of proline for an amino acid at position 2; a substitution of glutamine for an amino acid at position 71; or a substitution of proline for an amino acid at position 2 and a substitution of glutamine for an amino acid at position 71 in an amino acid sequence of SEQ ID NO: 8.

6. The pharmaceutical composition of claim 1, wherein the linker is a peptide linker consisting of 1 amino acid to 100 amino acids.

7. The pharmaceutical composition of claim 6, wherein the peptide linker consists of an amino acid sequence of [GS]x, [GGGS]x, or [GGGGS]x, wherein x is a natural number of 1 to 20.

8. The pharmaceutical composition of claim 7, wherein the peptide linker has an amino acid sequence of SEQ ID NO: 11.

9. The pharmaceutical composition of claim 1, wherein the neuropathy is polyneuropathy or small fiber neuropathy.

10. The pharmaceutical composition of claim 1, wherein the pharmaceutical composition has a protective effect on peripheral sensory nerves.

11. The pharmaceutical composition of claim 1, wherein the pharmaceutical composition inhibits vacuolation of dorsal root ganglion (DRG) cells.

12. The pharmaceutical composition of claim 1, wherein the administration frequency of the enzyme fusion protein to an individual in need thereof is reduced as compared to that of an enzyme other than the fusion protein.

13. The pharmaceutical composition of claim 1, wherein the pharmaceutical composition is administered to an individual once every two weeks or once a month.

14. The pharmaceutical composition of claim 1, wherein the enzyme fusion protein comprises a monomer including an amino acid sequence of SEQ ID NO: 13.

15. The pharmaceutical composition of claim 1, wherein X and X' are enzymes, each including an amino acid sequence the same as or different from each other.
